# EUROPEAN PATENT SPECIFICATION

(11) **EP 0 783 303 B1**
(45) Date of publication and mention of the grant of the patent: **27.11.2002**
(21) Application number: 95933137.2
(22) Date of filing: 15.09.1995
(51) Int. Cl.: A61K 31/05, A61K 31/03, A61P 35/00, A61P 9/10

(54) **USE OF AROMATIC HALIDES FOR TREATING MAMMALIAN CELL PROLIFERATION**
VERWENDUNG VON AROMATISCHEN HALOGENVERBINDUNGEN ZUR BEHANDLUNG DER ZELLPROLIFERATION IN SÄUGETIEREN
UTILISATION D'HALOGENURES AROMATIQUES POUR TRAITER LA PROLIFERATION CELLULAIRE MAMMALIENNE

(30) Priority: 16.09.1994 US 307887
(43) Date of publication of application: 16.07.1997
(73) Proprietor: PRESIDENT AND FELLOWS OF HARVARD COLLEGE, Cambridge, MA 02138 (US); CHILDREN'S MEDICAL CENTER CORPORATION, Boston Massachusetts 02115 (US)
(72) Inventor: HALPERIN, Jose, Brookline, MA 02146 (US); BRUGNARA, Carlo, Newton, MA 02161 (US)
(74) Representative: Jump, Timothy John Simon
(86) International application number: US9511821
(87) International publication number: WO96008240

(56) References cited:
- WO-A-87/07842
- WO-A-94/18967
- WO-A-94/18968
- ACTA PHARM.TURC., vol. 31, no. 2, 1989 pages 49-52, 'Studies on the stability of clotrimazole in simulated gastric and intestinal media'
- NETH.J.PLANT PATHOL., vol. 83, no. 1, 1977 pages 39-47,

## Description

### Field of the Invention

The invention relates to compounds for the inhibition of mammalian cell proliferation, including use in the treatment of cancer, arteriosclerosis and diseases characterized by neovascularization.

### Background of the Invention

Cell proliferation is a normal part of mammalian existence, necessary for life itself. Cell proliferation, however, is not always desirable and can be at the root of life-threatening conditions such as cancer, skin disease, inflammatory disease and arteriosclerosis.

Various imidazoles that are antimycotics have been discovered to be useful in inhibiting cell proliferation associated with cancer, angiogenic conditions and arteriosclerosis. These imidazoles include clotrimazol, miconazole and econazole. The use of clotrimazol, miconazole and econazole for inhibiting angiogenesis has been disclosed by Halperin *et al*. in WO 94/18967. The use of this particular class of imidazoles for inhibiting endothelial cell, vascular smooth muscle cell and fibroblast proliferation has been disclosed by Halperin, J. and Brugnara, C. in WO 94/18968. These imidazoles were characterized in part by their ability to inhibit the calcium activated potassium channel in erythrocytes. The imidazole group was common to all of the active compounds described and it was believed that the imidazole group was an essential component of the functionally active part of these molecules.

### Summary of the Invention

The present invention provides products for inhibiting mammalian cell proliferation. It has been discovered that the imidazole functionality of the compounds mentioned in the Background of the Invention is not required for the antiproliferation activity of such compounds. Thus, imidazoles that are antimycotics and that inhibit calcium activated potassium channel of erythrocytes have been modified for use in the present invention by removing the imidazole group. Such modified compounds typically have at least two aromatic groups separated from one another by not more than five atoms, and more typically not more than three atoms. The atoms joining the aromatic groups may be carbon, nitrogen and/or sulphur atoms. At least one of the aromatic groups is substituted with a chlorine or another functionality with an overall net negative charge.

According to one aspect of the invention, a compound is provided for use in the inhibition of unwanted mammalian cell proliferation. The compound has the following formula: 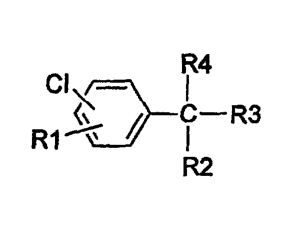 or pharmaceutically acceptable salts thereon wherein: R1 is selected from the group consisting of hydrogen, hydroxyl and halogen; R2 is selected from the group consisting of nothing, hydrogen, phenyl, and substituted phenyl, the substitution being a hydroxyl group; R3 is selected from the group consisting of hydrogen, hydroxyl, lower (1-3 carbon atoms) alkyl, and lower alkoxy; R4 is selected from the group consisting of sulfur-CH₂-R5, oxygen-CH₂-R5, =N-O-CH₂-R5, oxygen-phenyl-CH=CH₂, CH₂-CH(CH₃)-S-substituted phenyl, phenyl, and substituted phenyl, the substitution being selected from the group consisting of a hydroxyl group and a halogen; R5 is selected from the group consisting of vinyl, phenyl, halogen mono- substituted phenyl, halogen di-substituted phenyl, phenyl-S-phenyl, CH₂-O-phenyl, CH₂-O-halogen substituted phenyl, and: 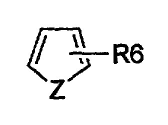 wherein Z is sulfur, oxygen, or nitrogen; and R6 is selected from the group consisting of hydrogen and halogen.

The preferred compounds are derivatives of clotrimazole that lack the imidazole group, derivatives of econazole that lack the imidazole group and derivatives of miconazole that lack the imidazole group.

The compounds are administrable to a subject that has a condition characterized by unwanted cell proliferation, including cancer, a noncancer angiogenic condition, or an arteriosclerotic condition.

According to another aspect of the invention, methods for inhibiting mammalian cell proliferation are provided. Mammalian cells are contacted with any one or more of the compounds described above in an amount effective to inhibit the proliferation of the mammalian cells. This aspect of the invention may be applied in vitro to arrest cell proliferation at a desired time. The mammalian cells treatable in this manner include vascular smooth muscle cells, fibroblasts, endothelial cells and various cancer cells.

The invention also provides pharmaceutical preparations that are sustained release implants containing the foregoing compounds, as well as pharmaceutical preparations that are cocktails of anticancer agents including the foregoing compounds.

These and other aspects of the invention will be described in greater details below.

### Detailed Description Of The Invention

The invention involves products for inhibiting mammalian cell proliferation. It previously has been established that certain imidazoles that are antimycotics and that inhibit the calcium activated potassium channel of erythrocytes can inhibit mammalian cell proliferation. The present invention involves the unexpected finding that metabolites and analogs of these imidazoles can inhibit mammalian cell proliferation, despite that fact that they do not contain an imidazole functionality.

The molecules useful according to the invention include molecules of the following general formula: 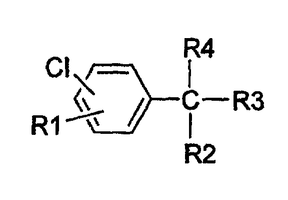 wherein R1 is selected from the group consisting of hydrogen, hydroxyl and halogen; wherein R2 is selected from the group consisting of nothing, hydrogen, phenyl, and substituted phenyl, the substitution being a hydroxyl group; wherein R3 is selected from the group consisting of hydrogen, hydroxyl, lower alkyl, and lower alkoxy; wherein R4 is selected from the group consisting of sulfur-CH₂-R5, oxygen-CH₂-R5, =N-O-CH₂-R5, oxygen-phenyl-CH=CH₂, 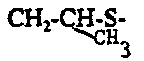 substituted phenyl, phenyl, and substituted phenyl, the substitution being selected from the group consisting of a hydroxyl group and a halogen; wherein R5 is selected from the group consisting of vinyl, phenyl, halogen mono-substituted phenyl, halogen di-substituted phenyl, phenyl-S-phenyl, CH₂-O-phenyl, CH₂-O-(halogen substituted) phenyl, and: 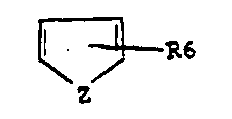 (wherein Z is sulfur, oxygen, or nitrogen); and R6 is selected from the group consisting of hydrogen and halogen.

The most preferred compounds are analogs of clotrimazole, miconazole and econazole, such molecules modified such that their imidazole group is replaced with a hydrogen, a hydroxyl, a lower (1-3 carbon atoms) alkyl or lower alkoxy group. Such compounds include: 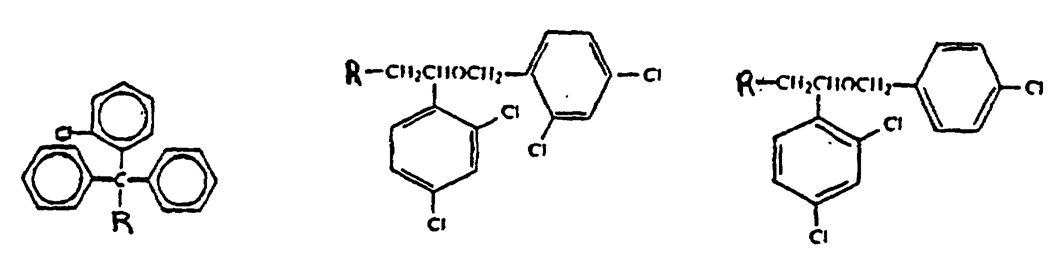 wherein R is hydrogen, hydroxyl, lower alkyl or lower alkoxy.

The preferred compounds are

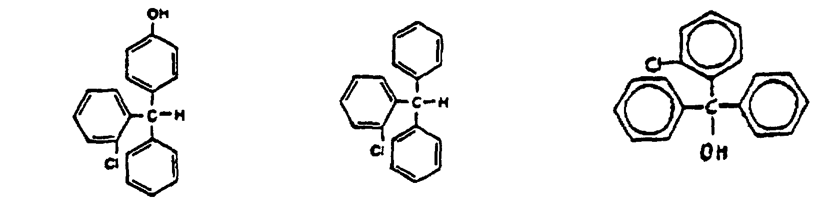

Other compounds believed useful according to the invention include derivatives of other imidazoles that are antimycotics and that inhibit the calcium activated potassium channel of erythrocytes including derivatives of oxiconazole, isoconazole, cloconazole, butoconazole, fenticonazole, enilconazole, omoconazole, sulconazole and tioconazole:

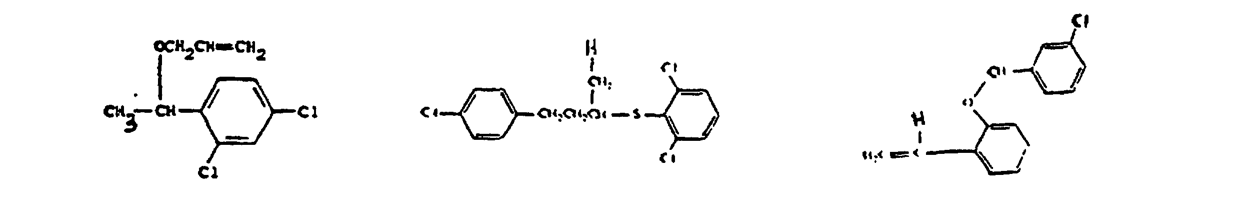

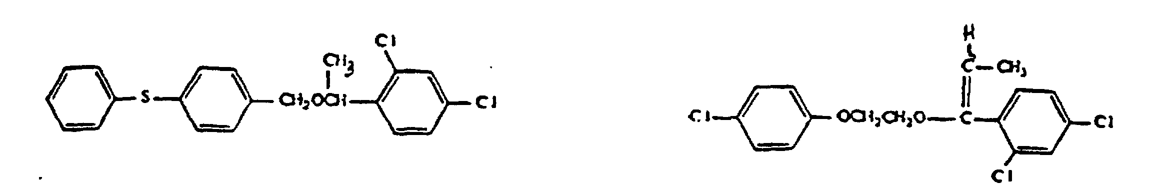

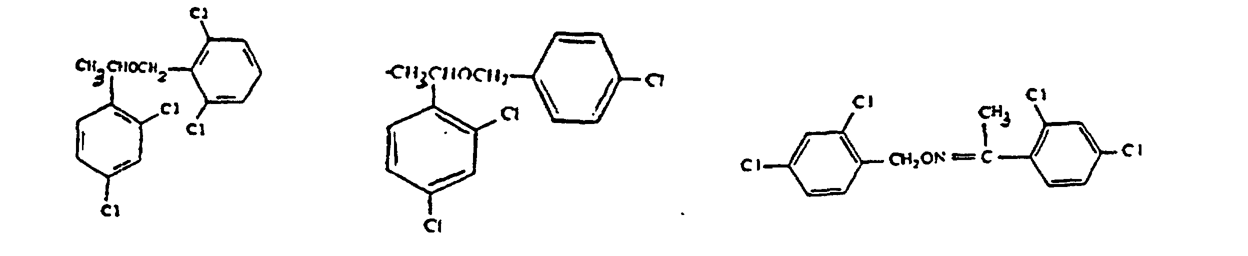

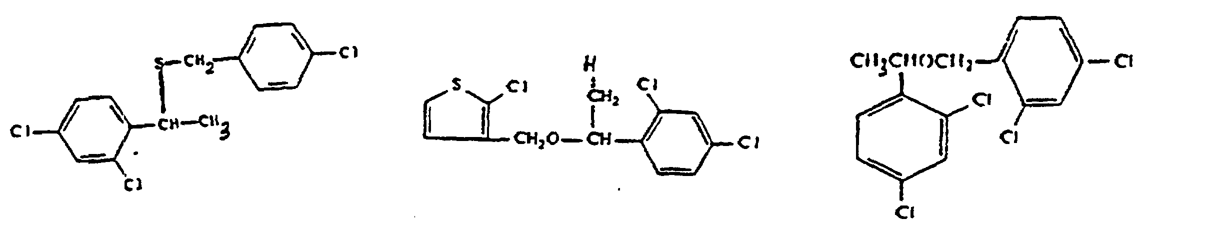

The foregoing compounds are derivatives of imidazoles that are commercially available. Such commercially available imidazoles may be modified according to conventional organic chemistry techniques to manufacture the foregoing compounds. Such compounds also may be synthesized de novo from readily available starting materials. The manufacture of these compounds is within the level of ordinary skill in the art.

The present invention is useful whenever it is desirable to inhibit mammalian cell proliferation in-situ, and desirably in the microcirculation of a human in need of such treatment. By definition, the word "in-situ" encompasses and includes the term "in vivo", "ex vivo", and "in vitro". Particular uses of the invention are described in the following paragraphs, these uses including prophylactic treatment and treatment of acute and chronic conditions.

### Cancer Treatments

It is believed that one third of the people in the United States will develop cancer. Cancer remains second only to cardiac disease as the main cause of death in the united States. Chemotherapy using the compounds of the invention is provided.
The invention is useful to treat various cancers including: biliary tract cancer; brain cancer, including glioblastomas and medelloblastomes; breast cancer; cervical cancer; choriocarcinoma; colon cancer; endometrial cancer; esophogeal cancer, gastric cancer, hematological neoplasms, including acute and chronic lymphocytic and myelogeneous leukemia, multiple myeloma, AIDS associated leukemias and adult T-cell leukemia lymphoma; intraepithelial neoplasms, including Bowen's disease and Paget's disease; liver cancer; lung cancer; lymphomas, including Hodgkin's disease and lymphozytic lymphomas; neuroblastomas; oral cancer, including squamous cell carcinoma; ovarian cancer, including those arising from epithelial cells, stromal cells, germ cells and mesenchymal cells; pancreas cancer; prostate cancer; rectal cancer; sarcomas, including leiomyosarcoma, rhabdomyosarcoma, liposarcoma, fibrosarcoma and osteosarcoma; skin cancer, including melanoma, Kaposi's sarcoma, basocellular cancer and squamous cell cancer; testicular cancer, including germinal tumors [seminoma, non-seminoma (teratomas, choriocarcinomas)], stromal tumors and germ cell tumors; thyroid cancer, including thyroid adenocarcinoma and medullar carcinoma; and renal cancer including adenocarcinoma and Wilms tumor.

The compounds useful in the invention may be delivered in the form of anti-cancer cocktails. An anti-cancer cocktail is a mixture of any one of the compounds useful with this invention with another anti-cancer drug and/or supplementary potentiating agent. The use of cocktails in the treatment of cancer is routine. In this embodiment, a common administration vehicle (e.g., pill, tablet, implant, injectable solution, etc.) would contain both the derivative of imidazole useful in this invention and the anti-cancer drug and/or supplementary potentiating agent.

Anti-cancer drugs include the derivatives of the imidazoles described above. Other anti-cancer drugs are well known and include: Aminoglutethimide; Asparaginase; Bleomycin; Busulfan; Carboplatin; Carmustine (BCNU); Chlorambucil; Cisplatin (cis-DDP); Cyclophosphamide; Cytarabine HCI; Dacarbazine; Dactinomycin; Daunorubicin HCI; Doxorubicin HCI; Estramustine phosphate sodium; Etoposide (V16-213); Floxuridine; Fluorouracil (5-FU); Flutamide; Hydroxyurea (hydroxycarbamide); Ifosfamide; Interferon Alfa-2a, Alfa 2b; Leuprolide acetate (LHRH-releasing factor analogue); Lomustine (CCNU); Mechlorethamine HCI (nitrogen mustard); Melphalan; Mercaptopurine; Mesna; Methotrexate (MTX); Mitomycin; Mitotane (o. p'-DDD); Mitoxantrone HCI; Octreotide; Plicamycin; Procarbazine HCI; Streptozocin; Tamoxifen citrate; Thioguanine; Thiotepa; Vinblastine sulfate; Vincristine sulfate; Amsacrine (m-AMSA); Azacitidine; Hexamethylmelamine (HMM); Interleukin 2; Mitoguazone (methyl-GAG; methyl glyoxal bis-guanylhydrazone; MGBG); Pentostatin; Semustine (methyl-CCNU); Teniposide (VM-26) and Vindesine sulfate.

Supplementary potentiating agents likewise are well characterized and include: tricyclic anti-depressant drugs (e.g., imipramine, desipramine, amitryptyline, clomipramine, trimipramine, doxepin, nortriptyline, protriptyline, amoxapine and maprotiline); non-tricyclic anti-depressant drugs (e.g., sertraline, trazodone and citalopram); Ca⁺⁺ antagonists (e.g., verapamil, nifedipine, nitrendipine and caroverine); Calmodulin inhibitors (e.g., prenylamine, trifluoroperazine and clomipramine); Amphotericin (an antifungal drug); perhexiline maleate (a drug used in the treatment of cardiovascular conditions); Triparanol analogues (e.g., tamoxifen); antiarrhythmic drugs (e.g., quinidine); antihypertensive drugs (e.g., reserpine); and Thiol depleters (e.g., buthionine and sulfoximine) and Tween 80 (a dispersing agent).

The compounds of the invention, when used in cocktails, are administrable in therapeutically effective amounts. A therapeutically effective amount will be determined by the parameters discussed above; but, in any event, is that amount which establishes a level of the drug in the area of the tumor which is effective in inhibiting the tumor growth.

The compounds of the invention may be used advantageously with other treatments that reduce the mass of the tumor. Depending upon the nature of the other treatment, the compounds of the invention are to be administered before, during or after treatment of the tumor. If surgical removal of as much as possible of the tumor is contemplated, then the compounds of the invention are to be administered before the surgery, during the surgery, and/or after the surgery. For example, they are administrable to the tumor by injection into the tumor mass prior to surgery in a single or several doses. The tumor (i.e. as much as possible of the tumor) then can be removed surgically. Further dosings of compounds to the site of the tumor (once removed) can be applied. Alternatively, surgical removal of as much as possible of the tumor can precede administration of the compounds to the tumor site.

### Arteriosclerosis

Arteriosclerosis is a term used to describe a thickening and hardening of the arterial wall. It is believed to be responsible for the majority of deaths in the United States and in most westernized societies. Atherosclerosis is one type of arteriosclerosis that is believed to be the cause of most coronary artery disease, aortic aneurysm and arterial disease of the lower extremities, as well as contributing to cerebrovascular disease. Atherosclerosis is the leading cause of death in the United States.

A normal artery typically is lined on its inner-side only by a single layer of endothelial cells, the intima. The intima overlays the media, which contains only a single cell type, the smooth muscle cell. The outer-most layer of the artery is the adventitia. With aging, there is a continuous increase in the thickness of the intima, believed to result in part from migration and proliferation of smooth muscle cells from the media. A similar increase in the thickness of the intima also occurs as a result of various traumatic events or interventions, such as occurs when a balloon dilatation procedure causes injury to the vessel wall. To date, there is no proven treatment for atherosclerosis.

The compounds of the invention inhibit endothelial cell, smooth muscle cell and fibroblast cell proliferation. Thus, methods for treating arteriosclerotic conditions and conditions involving fibrosis are provided.

The invention is used in connection with treating arteriosclerotic conditions. An arteriosclerotic condition as used herein means classical atherosclerosis, accelerated atherosclerosis, atherosclerosis lesions and any other arteriosclerotic conditions characterized by undesirable endothelial and/or vascular smooth muscle cell proliferation, including vascular complications of diabetes.

Proliferation of vascular smooth muscle cells is a main pathological feature in classical atherosclerosis. Liberation of growth factors from endothelial cells, it is believed, stimulates the proliferation of subintimal smooth muscle which, in turn, reduces the caliber and finally obstructs the artery. The invention is useful in inhibiting such proliferation and, therefore, in delaying the onset of, inhibiting the progression of or even halting the progression of such proliferation and the associated atherosclerotic condition.

Proliferation of vascular smooth muscle cells produces accelerated atherosclerosis which is the main reason for failure of heart transplants that are not rejected. This proliferation also is believed to be mediated by growth factors and can result ultimately in obstruction of the coronary arteries. The invention is useful in inhibiting such obstruction and reducing the risk of or even preventing such failures.

Vascular injury also can result in endothelial and vascular smooth muscle cell proliferation. The injury can be caused by any number of traumatic events, or interventions, including vascular surgery and angioplasty procedures performed for example by balloon dilatation catheters. Re-stenosis is the main complication of successful balloon angioplasties of the coronary arteries. It is believed to be caused by the release of growth factors as a result of mechanically injuring the endothelial cells lining the coronary arteries. The invention can be useful in inhibiting unwanted endothelial and smooth muscle cell proliferation and delaying or even avoiding altogether re-stenosis.

Other arteriosclerotic conditions include diseases of the arterial wall that include proliferation of endothelial and/or vascular smooth muscle cells, such as vascular complications of diabetes, diabetic glomerulosclerosis and diabetes retinopathy.

Other vascular conditions include by-pass surgery, coronary by-pass surgery, and procedures in addition to balloon angioplasty for re-establishing patency in occluded or partly occluded vessels, e.g. atherectomy, laser procedures and ultrasonic procedures.

### Angiogenic Conditions

Neovascularization, or angiogenesis, is the growth and development of new arteries. It is critical to the normal development of the vascular system, including injury-repair. There are, however, conditions characterized by abnormal neovascularization, including diabetic retinopathy, neovascular glaucoma, rheumatiod arthritis, and certain cancers. For example, diabetic retinopathy is a leading cause of blindness. There are two types of diabetic retinopathy, simple and proliferative. Proliferative retinopathy is characterized by neovascularization and scarring About one-half of those patients with proliferative retinopathy progress to blindness within about five years.

Another example of abnormal neovascularization is that associated with solid tumors. It is now established that unrestricted growth of tumors is dependant upon angiogenesis, and that induction of angiogenesis by liberation of angiogenic factors can be an important step in carcinogenesis. For example, basic fibroblast growth factor (bFGF) is liberated by several cancer cells and plays a crucial role in cancer angiogenesis. The demonstration that certain animal tumors regress when angiogenesis is inhibited has provided the most compelling evidence for the role of angiogenesis in tumor growth.

As used herein, an angiogenic condition means a disease or undesirable medical condition having a pathology including neovascularization. Such diseases or conditions include diabetic retinopathy, neovascular glaucoma and rheumatoid arthritis (noncancer angiogenic conditions). Cancer angiogenic conditions are solid tumors and cancers or tumors otherwise associated with neovascularization such as hemangioendotheliomas, hemangiomas and Kaposi's sarcoma.

Proliferation of endothelial and vascular smooth muscle cells is the main feature of neovascularization. The invention is useful in inhibiting such proliferation and, therefore, inhibiting or arresting altogether the progression of the angiogenic condition which depends in whole or in part upon such neovascularization. The invention is particularly useful when that condition has as an additional element endothelial or vascular smooth muscle cell proliferation that is not necessarily associated with the unwanted neovascularization For example, psoriasis may additionally involve endothelial cell proliferation that is independent of the endothelial cell proliferation associated with neovascularization. Likewise, a solid tumor which requires neovascularization for continued growth also may be a tumor of endothelial or vascular smooth muscle cells. In this case, the tumor cells themselves are inhibited from growing in the presence of the imidazole metabolites used in the invention.

### Other Proliferative Disorders

The invention is useful in inhibiting mammalian cell proliferation associated with virtually any proliferative disorder. In addition to the particular disorders mentioned above, the invention also is useful in treating other dermatologic diseases including keloids, hypertrophic scars, seborrheic keratosis, papilloma virus infection (e.g. producing verruca vulbaris, verruca plantaris, verruca plana, condilomata, etc.), actinic keratosis and eczema; other inflammatory diseases including proliferative glomerulonephritis; lupus erythematosus; scleroderma; temporal arthritis; thromboangiitis obliterans; mucocutaneous lymph node syndrome; and other pathologies mediated by growth factors including uterin leyomyomas.

The invention also is useful in treating fibrosis and other medical complications of fibrosis, all resulting in whole or in part from the proliferation of fibroblasts. Medical conditions involving fibrosis (other than atherosclerosis) include undesirable tissue adhesion resulting from surgery or injury.

### Formulations, Administration and Doses

When administered, the formulations of the invention are applied in pharmaceutically acceptable amounts and in pharmaceutically acceptable compositions. Such preparations may routinely contain salts, buffering agents, preservatives, compatible carriers, and optionally other therapeutic ingredients. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthalene-2-sulphonic, and benzene sulphonic. Also, pharmaceutically acceptable salts can be prepared as alkyline metal or alkyline earth salts, such as sodium, potassium or calcium salts of the carboxylic acid group.

Suitable buffering agents include: acetic acid and a salt (1-2% W/V); citric acid and a salt (1-3% W/V); boric acid and a salt (0.5-2.5% W/V); and phosphoric acid and a salt (0.8-2% W/V).

Suitable preservatives include benzalkonium chloride (0.003-0.03% W/V); chlorobutanol (0.3-0.9% W/V); parabens (0.01-0.25% W/V) and thimerosal (0.004-0.02% W/V).

The active compounds of the present invention may be a pharmaceutical composition having a therapeutically effective amount of an imidazole metabolite optionally included in a pharmaceutically-acceptable carrier. The term "pharmaceutically-acceptable carrier" as used herein means one or more compatible solid or liquid filler, dilutants or encapsulating substances which are suitable for administration to a human or other animal. The term "carrier" denotes an organic or inorganic ingredient, natural or synthetic, with which the active ingredient is combined to facilitate the application. The components of the pharmaceutical compositions also are capable of being comingled with the molecules of the present invention, and with each other, in a manner such that there is no interaction which would substantially impair the desired pharmaceutical efficacy.

Compositions suitable for parenteral administration conveniently comprise a sterile aqueous preparation of the active compound, which is preferably isotonic with the blood of the recipient. This aqueous preparation may be formulated according to known methods using those suitable dispersing or wetting agents and suspending agents. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic monoor di-glycerides. In addition, fatty acids such as oleic acid find use in the preparation of injectables. Carrier formulations suitable for oral, subcutaneous, intraveneous, intramuscular, etc. can be found in Remington's Pharmaceutical Sciences, Mack Publishing Company, Easton, PA.

The invention is used in connection with treating subjects having, suspected of having, developing or suspected of developing the conditions discussed above. A subject as used herein means humans, primates, horses, cows, pigs, sheep, goats, dogs, cats and rodents.

The compounds of the invention are to be administered in effective amounts. An effective amount means that amount necessary to delay the onset of, inhibit the progression of or halt altogether the onset or progression of the particular condition being treated. In general, an effective amount will be that amount necessary to inhibit mammalian cell proliferation in-situ.

When administered to a subject, effective amounts will depend, of course, on the particular condition being treated; the severity of the condition; individual patient parameters including age, physical condition, size and weight; concurrent treatment; frequency of treatment; and the mode of administration. These factors are well known to those of ordinary skill in the art and can be addressed with no more than routine experimentation. It is preferred generally that a maximum dose be used, that is, the highest safe dose according to sound medical judgment, particularly if acute sickle cell crises are the dominant clinical manifestation.

Target plasma levels will be those capable of inducing 75% inhibition in erythrocytes (see Examples). Dosage may be adjusted appropriately to achieve desired drug plasma levels. Generally, daily oral doses of active compounds will be from about 0.01 milligrams/kg per day to 1000 milligrams/kg per day. It is expected that oral doses in the range of 50 to 500 milligrams/kg, in one or several administrations per day, will yield the desired results. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds.

A variety of administration routes are available. The particular mode selected will depend of course, upon the particular drug selected, the severity of the disease state being treated and the dosage required for therapeutic efficacy. The methods of this invention, generally speaking, may be practiced using any mode of administration that is medically acceptable, meaning any mode that produces effective levels of the active compounds without causing clinically unacceptable adverse effects. Such modes of administration include oral, rectal, topical, nasal, transdermal or parenteral routes. The term "parenteral" includes subcutaneous, intraveneous, intramuscular, or infusion. Intraveneous and intramuscular routes are not particularly suited for long term therapy and prophylaxis. They could, however, be preferred in emergency situations. Oral administration will be preferred for prophylactic treatment because of the convenience to the patient as well as the dosing schedule.

The compositions may conveniently be presented in unit dosage form and may be prepared by any of the methods well known in the art of pharmacy. All methods include the step of bringing the active compounds into association with a carrier which constitutes one or more accessory ingredients. In general, the compositions are prepared by uniformly and intimately bringing the active compounds into association with a liquid carrier, a finely divided solid carrier, or both, and then, if necessary, shaping the product.

Compositions suitable for oral administration may be presented as discrete units such as capsules, cachettes, tablets, or lozenges, each containing a predetermined amount of the active compound. Other compositions include suspensions in aqueous liquors or non-aqueous liquids such as a syrup, an elixir, or an emulsion.

Other delivery systems can include time-release, delayed release or sustained release delivery systems. Such systems can avoid repeated administrations of the active compounds of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, diand triglycerides; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775 (Kent); 4,667,014 (Nestor et al.); and 4,748,034 and 5,239,660 (Leonard) and (b) diffusional systems in which an active component permeates at a controlled rate through a polymer, found in U.S. Patent Nos. 3,832,253 (Higuchi et al.) and 3,854,480 (Zaffaroni). In addition, a pump-based hardware delivery system can be used, some of which are adapted for implantation.

Use of a long-term sustained release implant may be particularly suitable for treatment of chronic conditions including, for example, cancer. "Long-term" release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above. Such implants can be particularly useful in treating solid tumors by placing the implant near or directly within the tumor, thereby affecting localized, high-doses of the compounds of the invention. Such implants can be especially useful in delivering compounds of the inventions that are not successfully ingested, or that do not pass biological barriers, such as the blood/brain barrier. They also can be used to avoid undesirable canulation, such as when brain tumors are being treated.

### Examples

### Materials

Abbreviations: ChTX, Charybdotoxin; CLT, clotrimazole; ECZ, econazole; MCZ, miconazole; FCZ, fluconazole; METZ, metronidazole; IbTX, iberotoxin; KTX, kaliotoxin; DIDS, di-isothiocyano-disulfonyl stilbene; hemoglobin concentration; MCHC, mean corpuscular hemoglobin concentration; MOPS, 3-[N-morpholino]propanesulfonic acid.

### Drugs and Chemicals

Synthetic charybdotoxin (ChTX) was purchased from Peptides International (Louisville, KY). A23187 was purchased from Calbiochem-Behring (LaJolla, CA). Fluconazole was provided by Pfizer Inc., Groton, CT, disulfonic acid (MOPS), clotrimazole (CLT), miconazole, econazole, metronidazole, and all other drugs and chemicals were purchased from Sigma Chemical Co. (St. Louis, MO) and Fisher Scientific Co. (Fair Lawn, NJ), and the radioisotope ⁸⁶Rb from Dupont (Billerica, MA)

### Assays for Cell Proliferation

DNA synthesis assessed by the uptake of [3H]thymidine: Cells are grown in either 48 or 96 wells plates (Costar, Cambridge, MA) at 10⁴ and 0.8 10³ cells per well, respectively, and grown in Dubelcco's modified Eagle's medium (DME, Gibco, Grand Island, NY) supplemented with 10% heat-inactivated calf serum; they are kept at 37°C in 5% CO₂. When they reach confluence, usually between 3 and 4 days, the medium is replaced with DME 0.5% serum to make them quiescent, and mitogenesis assays are performed 24 hours later.

Quiescent cells are exposed to a mitogenic stimulus, such as 10% serum, PDGF (Sigma Co. St. Louis, Mo), bFGF (Upstate Biotechnologies, Lake Placid, NY), or other appropriate mitogen according to the cell line, and 3 hours later 1 µCi/ml of [3H]thymidine (Dupont, Billerica, MA) is added to the wells, and the cells maintained at 37°C/5% CO2 for additional 21 hours. Then the cells are washed 3 times with DME medium and the acid-precipitable radioactivity is extracted with cold 10% TCA (Sigma, Co). After neutralization with 0.3 N NaOH (Sigma Co.), aliquots are counted in a Packard Tri-Carb Scintillation counter (Packard Instrument, Downer's Grove, IL).

Measurement of cell density in culture plates: Cells of a specific test cell line are seeded at precisely the same low density in culture plates and incubated for approximately 12 hours in DME 10% serum, or other culture medium depending on the cell line tested. After 12 hours, the test drug, for example clotrimazole 10 µM, is added to the cell medium of one plate and a similar amount of only the carrier of the drug, for example ethanol 10µl, to another plate. After 48 to 74 hours, the cell density in control (ethanol) and experimental plates is assessed under a light inverted microscope, by measuring the surface of the culture plate covered by the cell monolayer. Alternatively, the cells can be detached from the plate by incubation with trypsin (Sigma, Co.) 50% (v/v) in ethylene diaminotetraacetic acid (ECTA; Sigma, Co); then the cells are counted in an hemocytometer chamber (Fisher, Pittsburgh, PA).

### Assays for Inhibitors of Ca⁺⁺ Activated K Channel

Ca⁺⁺-sensitive K⁺ channels have wide distribution among cells, including the human red cell where they were originally discovered and which is the most commonly utilized assay system for activators and inhibitors of the channel for the following reasons: they are readily available, can be easily manipulated in the laboratory, and transport assays can be accurately standardized by reading the hemoglobin concentration of a red cell suspension

Preparation of Human Red Blood Cells: Blood is collected in heparinized tubes and centrifuged in a Sorvall centrifuge (RB 5B, Du Pont Instruments, Newtown, CT) at 5°C for 10 minutes at 3000 g. Plasma and buffy coat are carefully removed and the cells washed four times with a washing solution containing 150 mM choline chloride (Sigma Co), 1mM MgCl₂ (Sigma Co), 10mM Tris-MOPS (Sigma, Ca), pH 7.4 at 4°C(CWS). An aliquot of cells is then suspended in an approximately equal volume of CWS, and from this original cell suspension hematocrit (Hct) and hemoglobin (optical density at 540 nm) are determined.

Methods to Test Inhibitors of the Ca⁺⁺ Activated K: To test inhibitors of the Ca⁺⁺ activated K channel, the channel is activated using the calcium ionophore A23187 (Chalbiochem).

By Atomic Absorption Spectrometry: Washed human erythrocyte are suspended at an hematocrit ≅ 1% in CWS containing 0.150mM CaCl₂ (Sigma Co). Aliquots of 1 ml are removed at 0, 3 and 5 minutes, layered on top of 0.3ml of the oil n-butyl phthalate (Fair Lane, NJ) placed in an Eppendorf microtube (Fisher) and then centrifuged in a micro centrifuge for 20 seconds. At time 5.30 minutes, ionophore A23187 (1µM final concentration) is added and samples removed and spin down through phthalate at times 6, 7, 8 and 9 minutes. The supernatant on top of the oil layer is removed and its K⁺ concentration is measured by atomic absorption spectrometry using a Perking Elmer model 5000 spectrometer (Perkin Elmer Corp., Norwolk, CT). The efflux of K+ (mmol/l cells/h) in the absence and presence of the inhibitor is calculated from the slope of the curves relating the K⁺ concentration in the supernatants (mmol/l cells) vs. time (min.).

By radioisotopic measurement of ⁸⁶Rb influx. The incubation medium is the same but contains 2 mM KCl and 1 µCi/ml of the radioactive tracer ⁸⁶Rb. After spinning the samples through the phthalate layer, the tubes are rapidly frozen (-80°C) by immersion in methanol-dry ice, the tips of the tubes containing the packed red cells cut, and counted in a Packard Gamma Counter.

### Example 1

The inhibitory effect of clotrimazole metabolite B (2-chlorophenyl-bis-phenyl-methanol) on the Ca⁺⁺-activated K channel of human erythrocytes was assessed in the presence of 60 µmol A231H7/L cell and 100 µMCaCl₂. This metabolite markedly inhibited the Ca⁺⁺-activated 86Rb influx and K efflux. Mean values of IC₅₀ was about .9 mM.

### Example 2

The ability of metabolite B to inhibit Ca⁺⁺-activated potassium transport was measured also by evaluating displacement of ¹²⁵I-ChTX. It previously has been shown (Brugnara, J.Biol.Chem. 1993) for ChTX that there is a two to three fold increase in the IC₅₀ value for displacement of ¹²⁵I-ChTX by ChTX compared with the inhibition of K transport by ChTX. The percent inhibit of K transport was greater than 50% when cells were treated with 1µM metabolite B and reached maximal levels at 10 µM metabolite B.

### Example 3

The inhibitory effect of metabolite B on cell proliferation was assessed in Swiss 3T3 cells (murine fibroblast cell line). Quiescent cells were stimulated with purified growth factors (bFGF) and synthesis of DNA was assessed by the incorporation of [3H]thymidine measured 24 hours later. 10 µM CLT completely inhibited bFGF stimulated DNA synthesis. Metabolite B, ketoprofen and diclofenac also inhibited cell proliferation, with ketoprofen inhibiting thimidine update by about 80% and CLT metabolite B and diclofenac inhibiting thimidine uptake by about 50% over controls.

### Example 4

A line of human melanoma cells (MM RU) was used in this experiment. The MM RU cell line produces metasteses only in the lungs (Byers, H.R., et al., "Organ Specific Metasteses in ImmunoDeficient Mice Injected with Human Melanoma cells: A Quantitative Pathologic Analysis. Melanoma Res. 3:247-253 (1993)). These cells were cultured in the presence and absence of CLT metabolite B and thimidine uptake was measured. 10µM of CLT metabolite B inhibited thimidine uptake by about 60% versus controls.

### Example 5

A line of colon adenocarcinoma (HT59) cells was used in this experiment. The cells were cultured in the presence and absence of CLT metabolite B. 10µM of CLT metabolite B inhibited thimidine uptake by approximately 50% versus controls.

Those skilled in the art will be able to ascertain with no more than routine experimentation numerous equivalents to the specific imidazoles and processes described herein. Such equivalents are considered to be within the scope of the invention and are intended to be embraced by the following claims in which we claim:

## Claims

1. A compound having the formula: 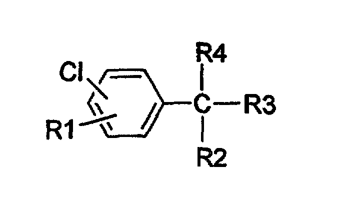 or a pharmaceutically acceptable salt thereof, wherein:
R1 is selected from the group consisting of hydrogen, hydroxyl and halogen;
R2 is selected from the group consisting of nothing, hydrogen, phenyl, and substituted phenyl, the substitution being a hydroxyl group;
R3 is selected from the group consisting of hydrogen, hydroxyl, C1-C3 alkyl, and C1-C3 alkoxy;
R4 is selected from the group consisting of sulfur-CH₂-R5, oxygen-CH₂-R5, =N-O-CH₂-R5, oxygen-phenyl-CH=CH₂, CH₂-CH(CH₃)-S- substituted phenyl, phenyl, and substituted phenyl, the substitution being selected from the group consisting of a hydroxyl group and a halogen;
R5 is selected from the group consisting of vinyl, phenyl, halogen mono-substituted phenyl, halogen di-substituted phenyl, phenyl-S-phenyl, CH₂-O-phenyl, CH₂-O-halogen substituted phenyl, and: 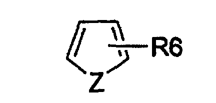
wherein Z is sulfur, oxygen, or nitrogen; and R6 is selected from the group consisting of hydrogen and halogen,
for use in the inhibition of unwanted mammalian cell proliferation.

2. A compound according to claim 1, wherein said compound is selected from a group of compounds: 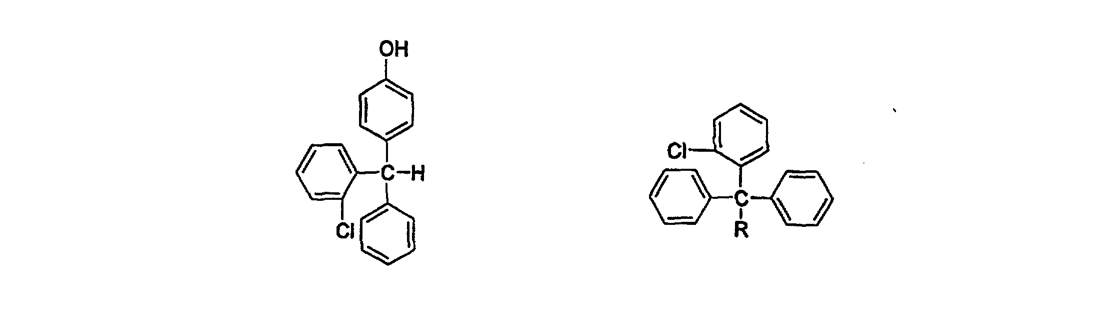 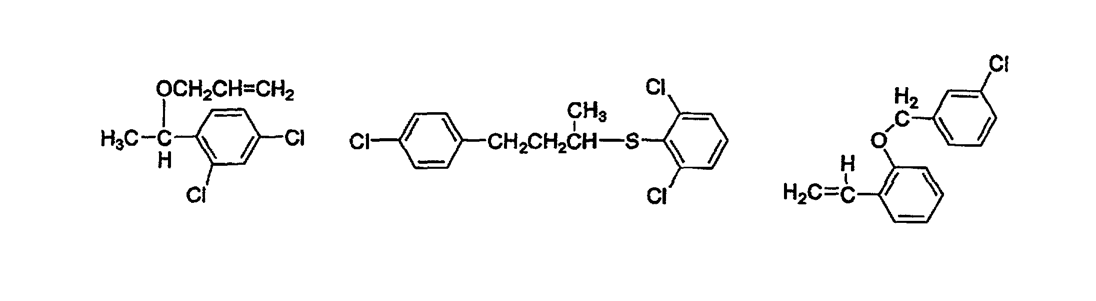 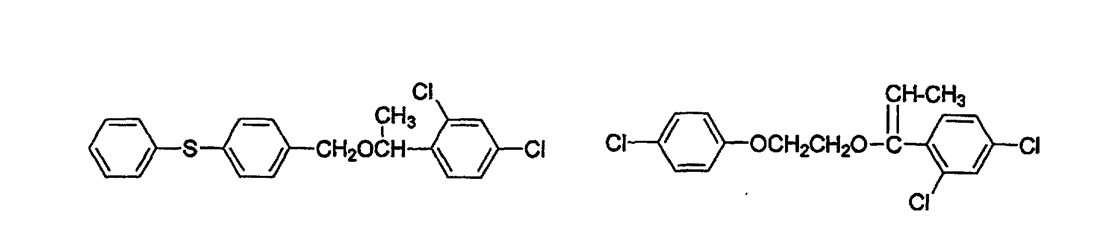 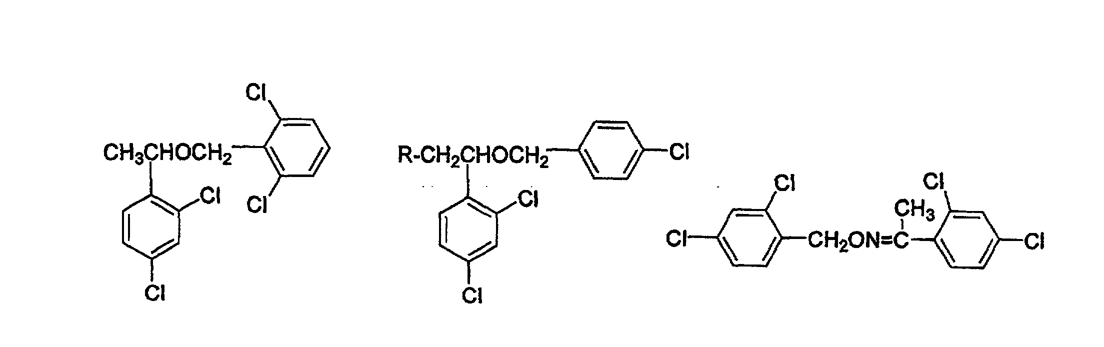 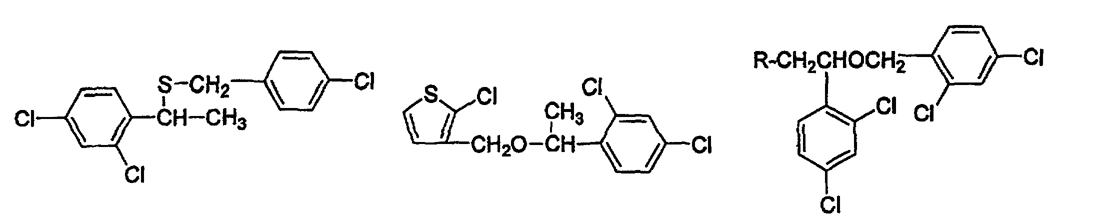 wherein R is hydrogen, hydroxyl, C1-C3 alkyl or C1-C3 alkoxy.

3. A compound according to claim 2, wherein said compound is selected from a group of compounds:

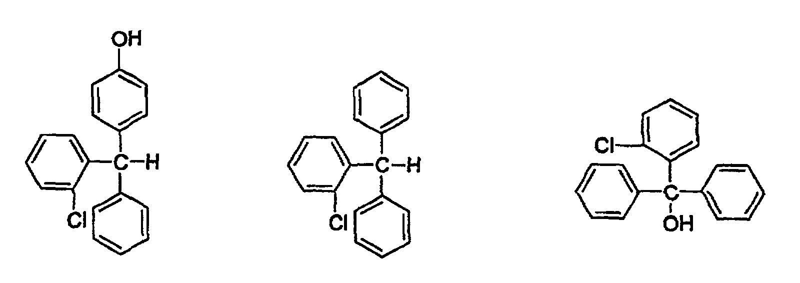

4. A compound according to claim 3, wherein the compound is:

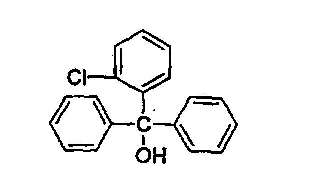

5. A compound according to claim 2, wherein said compound is:

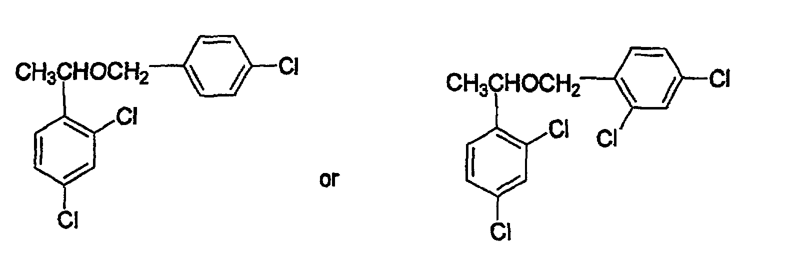

6. A compound according to any one of the preceding claims, wherein the mammalian cells are selected from the group consisting of vascular smooth muscle cells, fibroblasts, endothelial cells and cancer cells.

7. Use of a compound according to any of claims 1-5 in the manufacture of a medicament for treatment of a condition **characterized by** unwanted mammalian cell proliferation.

8. A use according to claim 7, wherein the unwanted mammalian cell proliferation is cancer, an angiogenic condition, an arteriosclerotic condition, a dermatologic disease, an inflammatory disease, or fibrosis.

9. A use according to claim 8, wherein said cancer is selected from the group consisting of: biliary tract cancer; brain cancer; breast cancer; cervical cancer; choriocarcinoma; colon cancer; endometrial cancer; esophogeal cancer; gastric cancer; hematological neoplasms; intraepithelial neoplasms; liver cancer; lung cancer; lymphomas; neuroblastomas; oral cancer; ovarian cancer; pancreas cancer; prostate cancer; rectal cancer; sarcomas; skin cancer; testicular cancer; thyroid cancer; and renal cancer.

10. A use according to claim 9, wherein said cancer is skin cancer.

11. A use according to claim 9, wherein said cancer is colon cancer.

12. A use according to claim 8, wherein the said arteriosclerotic condition is selected from the group consisting of atherosclerosis and vascular complications of diabetes.

13. A use according to claim 8, wherein said angiogenic condition is selected from the group consisting of: diabetic retinopathy; neovascular glaucoma; rheumatoid arthritis, a non cancer angiogenic condition; solid tumors, a cancer angiogenic condition; hemangioendotheliomas; hemangiomas; Kaposi's sarcoma and psoriasis.

14. A use according to claim 8, wherein said dermatologic disease is selected from the group consisting of: keloids; hypertrophic scars; seborrheic keratosis; papilloma virus infection; actinic keratosis and eczema.

15. A use according to claim 8, wherein said inflammatory disease is selected from the group consisting of: proliferative glomerulonephritis; lupus erythematosus; scleroderma; temporal arthritis; thromboangiitis obliterans; mucocutaneous lymph node syndrome and uterin leyomyomas.

16. A pharmaceutical preparation comprising sustained release implant containing a compound having the formula: 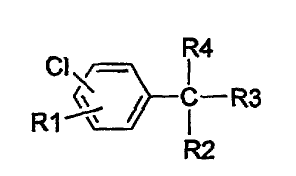 or a pharmaceutically acceptable salt thereof, wherein:
R1 is selected from the group consisting of hydrogen, hydroxyl and halogen;
R2 is selected from the group consisting of nothing, hydrogen, phenyl, and substituted phenyl, the substitution being a hydroxyl group;
R3 is selected from the group consisting of hydrogen, hydroxyl, C1-C3 alkyl, and C1-C3 alkoxy;
R4 is selected from the group consisting of sulfur-CH₂-R5, oxygen-CH₂-R5, =N-O-CH₂-R5, oxygen-phenyl-CH=CH₂, CH₂-CH(CH₃)-S- substituted phenyl, phenyl, and substituted phenyl, the substitution being selected from the group consisting of a hydroxyl group and a halogen;
R5 is selected from the group consisting of vinyl, phenyl, halogen mono substituted phenyl, halogen di-substituted phenyl, phenyl-S-phenyl, CH₂-O-phenyl, CH₂-O-halogen substituted phenyl, and: 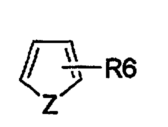
wherein Z is sulfur, oxygen, or nitrogen; and R6 is selected from the group consisting of hydrogen and halogen.

17. A pharmaceutical preparation comprising a cocktail of anticancer agents including a compound having the formula: 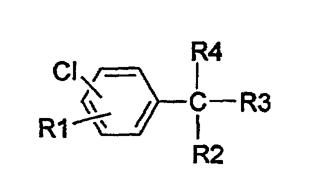 or a pharmaceutically acceptable salt thereof, wherein:
R1 is selected from the group consisting of hydrogen, hydroxyl and halogen;
R2 is selected from the group consisting of nothing, hydrogen, phenyl, and substituted phenyl, the substitution being a hydroxyl group;
R3 is selected from the group consisting of hydrogen, hydroxyl, C1-C3 alkyl, and C1-C3 alkoxy;
R4 is selected from the group consisting of sulfur-CH₂-R5, oxygen-CH₂-R5, =N-O-CH₂-R5, oxygen-phenyl-CH=CH₂, CH₂-CH(CH₃)-S- substituted phenyl, phenyl, and substituted phenyl, the substitution being selected from the group consisting of a hydroxyl group and a halogen;
R5 is selected from the group consisting of vinyl, phenyl, halogen mono substituted phenyl, halogen di-substituted phenyl, phenyl-S-phenyl, CH₂-O-phenyl, CH₂-O-halogen substituted phenyl, and: 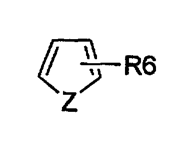
wherein Z is sulfur, oxygen, or nitrogen; and R6 is selected from the group consisting of hydrogen and halogen.

18. A pharmaceutical preparation according to claim 16 or 17, wherein the compound is selected from the group: 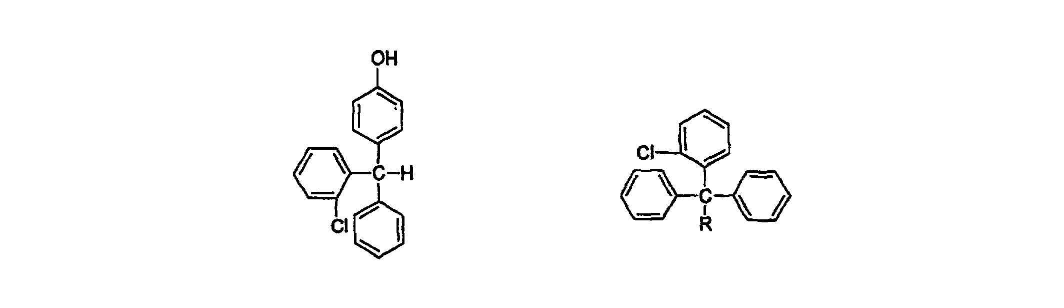 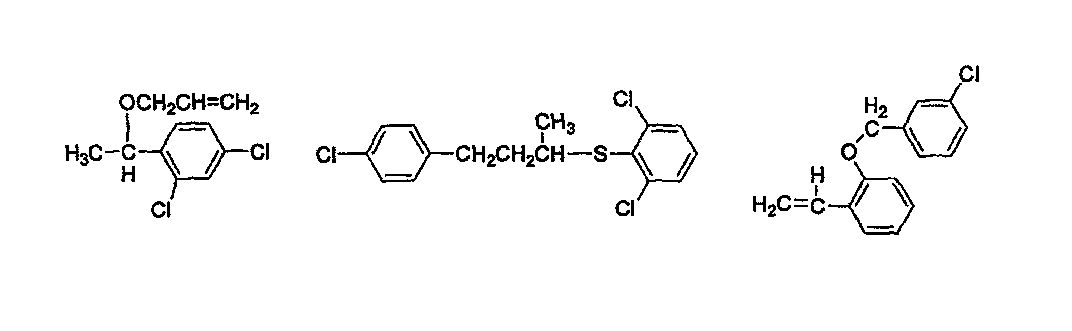 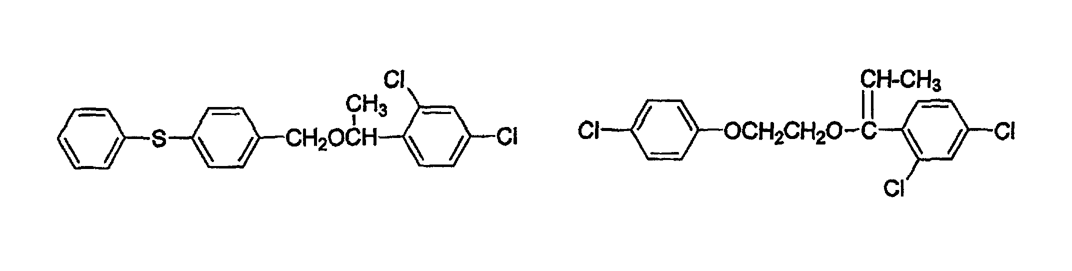 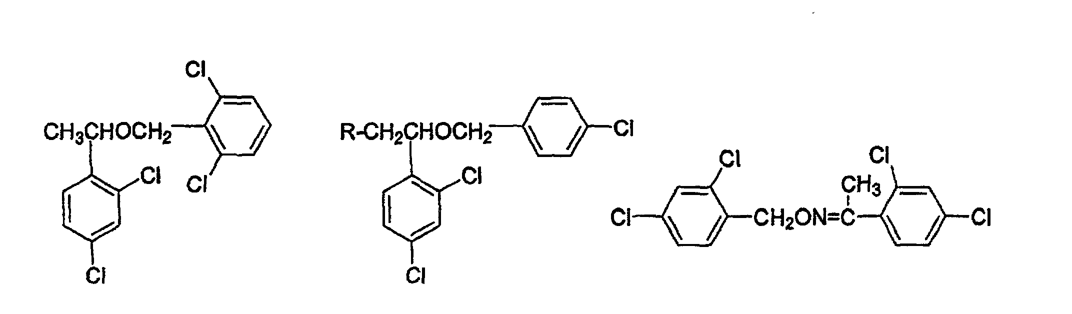 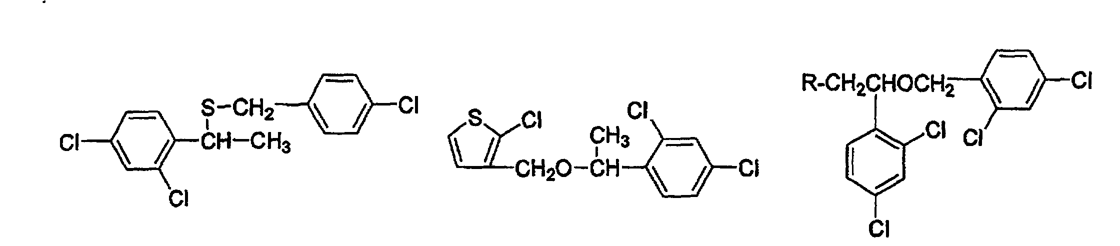 wherein R is hydrogen, hydroxyl, C1-C3 alkyl or C1-C3 alkoxy.

19. A pharmaceutical preparation according to claim 18, wherein the compound is selected from the group:

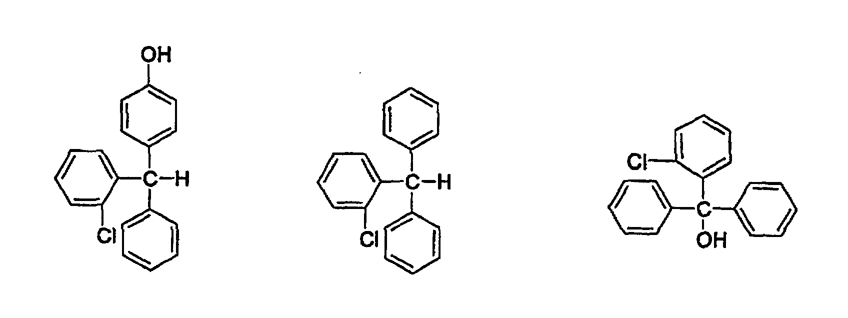

20. A pharmaceutical preparation of claim 19, wherein the compound is:

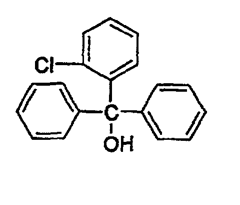

21. A pharmaceutical preparation according to claim 18, wherein the compound is:

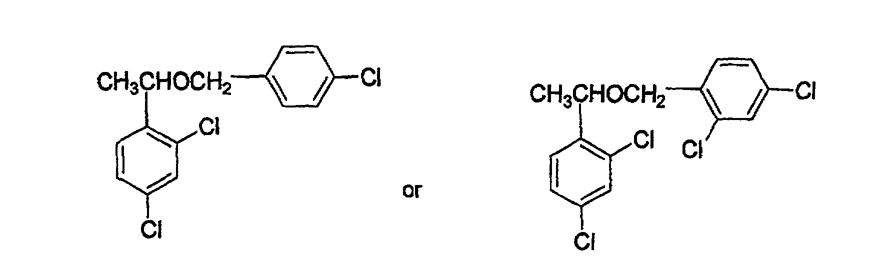

## Patentansprüche

1. Eine Verbindung mit der Formel 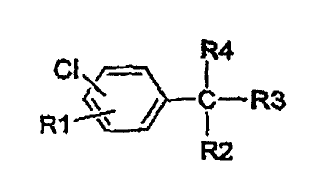 oder einem pharmazeutisch akzeptablen Salz davon, wobei:
R1 aus der Gruppe ausgewählt ist, die aus Wasserstoff, Hydroxyl und Halogen besteht;
R2 aus der Gruppe ausgewählt ist, die aus nichts, Wasserstoff, Phenyl und substituiertem Phenyl besteht, wobei die Substitution aus einer Hydroxylgruppe besteht;
R 3 aus der Gruppe ausgewählt ist, die aus Wasserstoff, Hydroxyl, C1-C3-Alkyl und C1-C3-Alkoxy besteht;
R4 aus der Gruppe ausgewählt ist, die aus Schwefel-CH₂-R5, Sauerstoff-CH₂-R5, =N-O-CH₂-R5, Sauerstof-Phenyl-CH=CH₂, CH₂-CH(CH₃)-S-substituiertes Phenyl, Phenyl und substituiertes Phenyl besteht, wobei die Substitution aus der Gruppe ausgewählt ist, die aus einer Hydroxylgruppe und einem Halogen besteht;
R5 aus der Gruppe ausgewählt ist, die aus Vinyl, Phenyl, Halogen monosubstituiertem Phenyl, Halogen di-substituiertem Phenyl, Phenyl-S-Phenyl, CH₂-O-Phenyl, CH₂-O-Halogen-substituiertem Phenyl und: 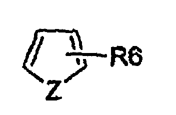 besteht, wobei Z aus Schwefel, Sauerstoff oder Stickstoff besteht und R6 aus der Gruppe ausgewählt ist, die aus Wasserstoff und Halogen besteht,
zur Verwendung bei der Verhinderung einer unerwünschten Vermehrung von Säugetierzellen.

2. Eine Verbindung gemäß Anspruch 1, wobei die besagte Verbindung ausgewählt ist aus einer Gruppe von Verbindungen: 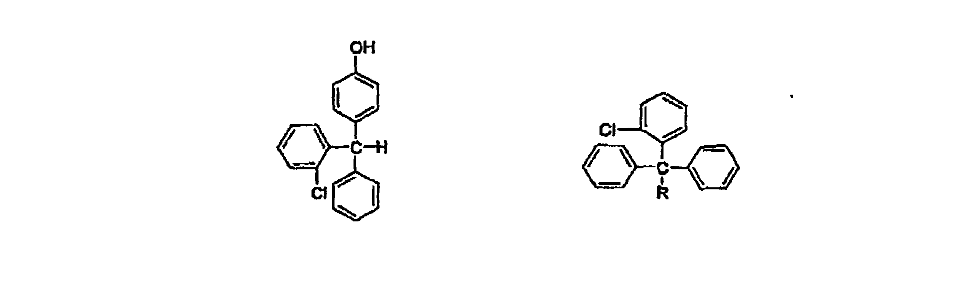 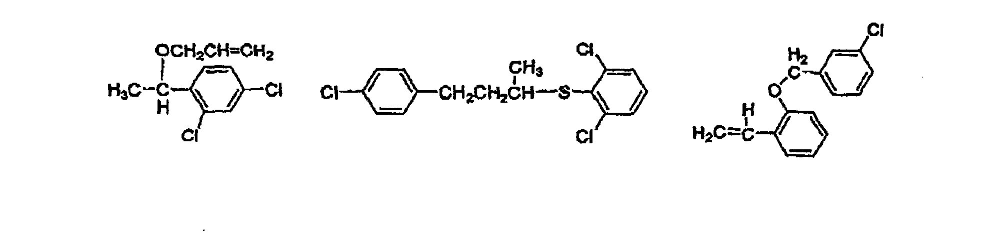 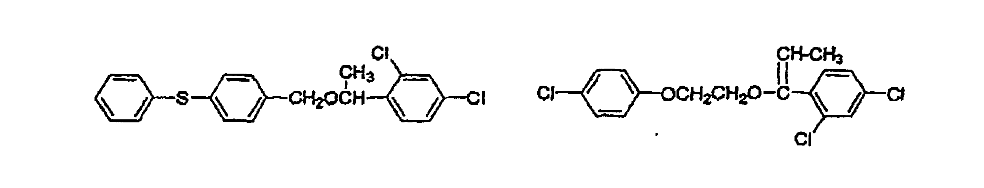 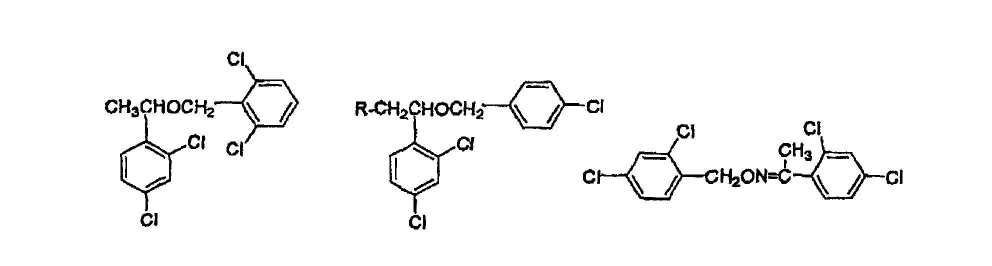 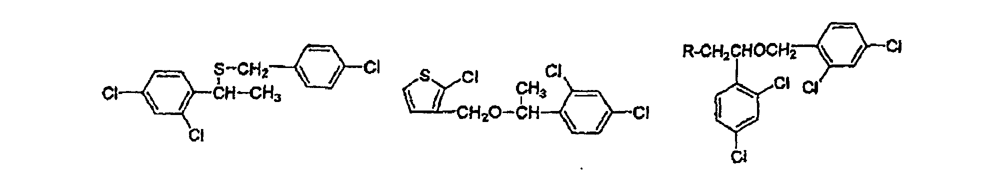 wobei R aus Wasserstoff, Hydroxyl, C1-C3-Alkyl oder C1-C3-Alkoxy besteht.

3. Eine Verbindung gemäß Anspruch 2, wobei die besagte Verbindung ausgewählt ist aus einer Gruppe von Verbindungen:

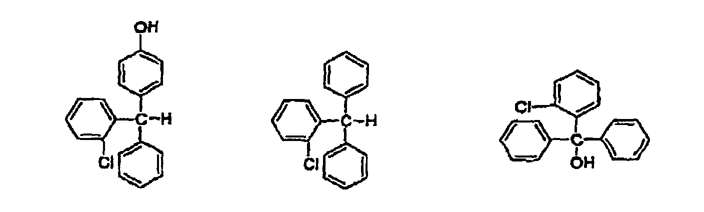

4. Eine Verbindung gemäß Anspruch 3, wobei die Verbindung aus 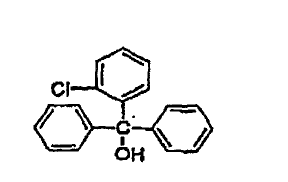 besteht.

5. Eine Verbindung gemäß Anspruch 2, wobei die Verbindung aus 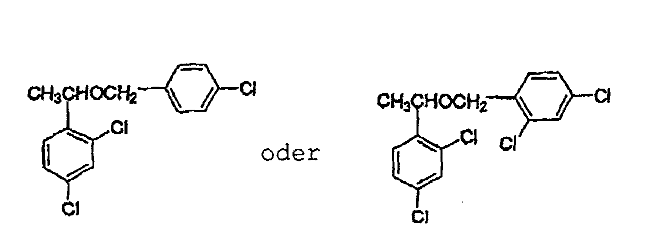 besteht.

6. Eine Verbindung gemäß einem der vorangehenden Ansprüche, wobei die Säugetierzellen aus der Gruppe ausgewählt sind, die aus glatten Muskelgefäßzellen, Fibroblasten, Endothelzellen und Krebszellen besteht.

7. Die Verwendung einer Verbindung gemäß einem der Ansprüche 1 - 5 bei der Herstellung eines Medikamentes zur Behandlung eines Zustandes, der durch die unerwünschte Vermehrung von Säugetierzellen gekennzeichnet ist.

8. Eine Verwendung gemäß Anspruch 7, wobei die unerwünschte Vermehrung von Säugetierzellen durch Krebs, einen angiogenen Zustand, einen arteriosklerotischen Zustand, eine dermatologische Erkrankung, eine entzündliche Erkrankung oder durch eine Fibrose hervorgerufen wird.

9. Eine Verwendung gemäß Anspruch 8, wobei der besagte Krebs aus der Gruppe ausgewählt ist, die aus Krebs des Gallenblasentraktes; Gehirntumor, Brustkrebs, Cervicalkarzinom, Chorionkarzinom; Dickdarmkrebs; Endometriumkarzinom; Speiserörenkrebs; Magenkrebs; hematologische Neoplasmen; intraepitheliale Neoplasmen; Leberkrebs; Lungenkrebs; Lymphome; Neuroblastome; Mundkrebs; Ovarialkarzinom; Pancreaskarzinom, Prostatakarzinom; Rektalkarzinom; Sarkome; Hautkrebs, Hodenkrebs; Schilddrüsenkrebs und Nierenkrebs besteht.

10. Eine Verwendung gemäß Anspruch 9, wobei der besagte Krebs Hautkrebs ist.

11. Eine Verwendung gemäß Anspruch 9, wobei der besagte Krebs Dickdarmkrebs ist.

12. Eine Verwendung gemäß Anspruch 8, wobei der besagte arteriosklerotische Zustand aus der Gruppe ausgewählt ist, die aus Atherosklerose und Gefäßkomplikationen bei Diabetes besteht.

13. Eine Verwendung gemäß Anspruch 8, wobei der besagte angiogenen Zustand aus der Gruppe ausgewählt ist, die aus diabetischer Retinopathie; neovaskulärem Glaukom; rheumatischer Arthritis, einem gutartigen angiogenen Zustand; festen Tumoren, einem bösartigen angiogenen Zustand; Hämangioendotheliom; Hämangiom; Kaposisarkom und Psoriasis besteht.

14. Eine Verwendung gemäß Anspruch 8, wobei die besagte dermatologische Erkrankung aus der Gruppe ausgewählt ist, die aus Keloiden; hypertrophischen Narben; seborrheischer Keratose; Papillom-Virusinfektion, aktinischer Keratose und Ekzemen besteht.

15. Eine Verwendung gemäß Anspruch 8, wobei die besagte entzündliche Erkrankung aus der Gruppe ausgewählt ist, die aus proliferativer Glomerulonephritis; Lupus erythematosus; Sklerodermie; temporärer Arthritis; Thromboangiitis obliterans; mucocutanes Lymphknotensyndrom und Gebärmutter-Leiomyom besteht.

16. Eine pharmazeutische Zubereitung mit einem Implantat mit verzögerter Freisetzung, das eine Verbindung der Formel: 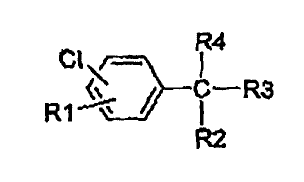 oder ein pharmazeutisch akzeptables Salz davon enthält, wobei:
R1 aus der Gruppe ausgewählt ist, die aus Wasserstoff, Hydroxyl und Halogen besteht;
R2 aus der Gruppe ausgewählt ist, die aus nichts, Wasserstoff, Phenyl und substituiertem Phenyl besteht, wobei die Substitution aus einer Hydroxylgruppe besteht;
R 3 aus der Gruppe ausgewählt ist, die aus Wasserstoff, Hydroxyl, C1-C3-Alkyl und C1-C3-Alkoxy besteht;
R4 aus der Gruppe ausgewählt ist, die aus Schwefel-CH₂-R5, Sauerstoff-CH₂-R5, =N-O-CH₂-R5, Sauerstof-Phenyl-CH=CH₂, CH₂-CH(CH₃)-S-substituiertes Phenyl, Phenyl und substituiertes Phenyl besteht, wobei die Substitution aus der Gruppe ausgewählt ist, die aus einer Hydroxylgruppe und einem Halogen besteht;
R5 aus der Gruppe ausgewählt ist, die aus Vinyl, Phenyl, Halogen monosubstituiertem Phenyl, Halogen di-substituiertem Phenyl, Phenyl-S-Phenyl, CH₂-O-Phenyl, CH₂-O-Halogen-substituiertem Phenyl und: 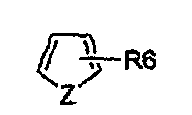 besteht, wobei Z aus Schwefel, Sauerstoff oder Stickstoff besteht und R6 aus der Gruppe ausgewählt ist, die aus Wasserstoff und Halogen besteht.

17. Eine pharmazeutische Zubereitung mit einer Mischung von krebshemmenden Substanzen, die eine Verbindung der Formel: 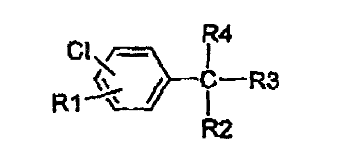 oder ein pharmazeutisch akzeptables Salz davon enthält, wobei:
R1 aus der Gruppe ausgewählt ist, die aus Wasserstoff, Hydroxyl und Halogen besteht;
R2 aus der Gruppe ausgewählt ist, die aus nichts, Wasserstoff, Phenyl und substituiertem Phenyl besteht, wobei die Substitution aus einer Hydroxylgruppe besteht;
R 3 aus der Gruppe ausgewählt ist, die aus Wasserstoff, Hydroxyl, C1-C3-Alkyl und C1-C3-Alkoxy besteht;
R4 aus der Gruppe ausgewählt ist, die aus Schwefel-CH₂-R5, Sauerstoff-CH₂-R5, =N-O-CH₂-R5, Sauerstof-Phenyl-CH=CH₂, CH₂-CH(CH₃)-S-substituiertes Phenyl, Phenyl und substituiertes Phenyl besteht, wobei die Substitution aus der Gruppe ausgewählt ist, die aus einer Hydroxylgruppe und einem Halogen besteht;
R5 aus der Gruppe ausgewählt ist, die aus Vinyl, Phenyl, Halogen monosubstituiertem Phenyl, Halogen di-substituiertem Phenyl, Phenyl-S-Phenyl, CH₂-O-Phenyl, CH₂-O-Halogen-substituiertem Phenyl und: 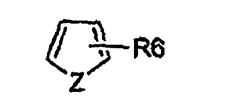 besteht, wobei Z aus Schwefel, Sauerstoff oder Stickstoff besteht und R6 aus der Gruppe ausgewählt ist, die aus Wasserstoff und Halogen besteht.

18. Eine pharmazeutische Zubereitung gemäß Anspruch 16 oder 17, wobei die Verbindung ausgewählt ist aus der Gruppe: 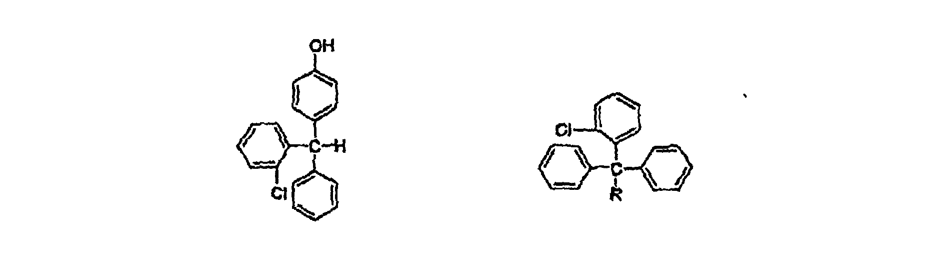 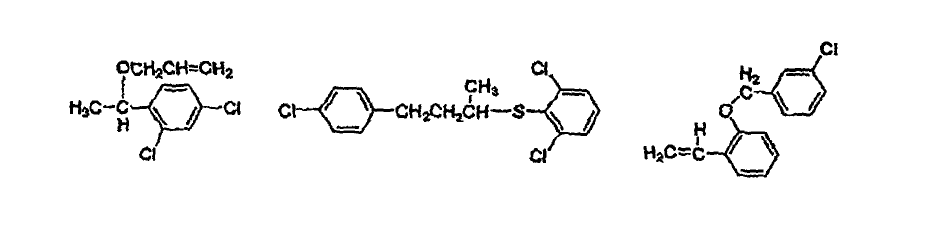 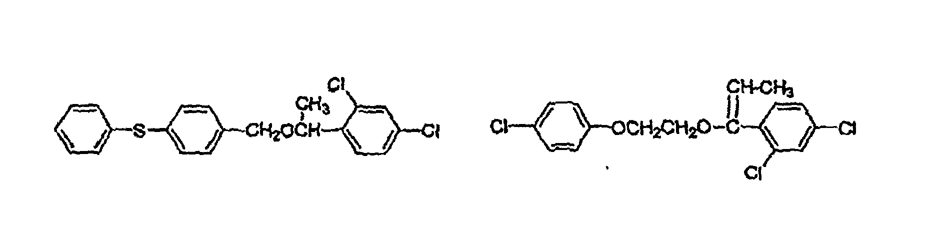 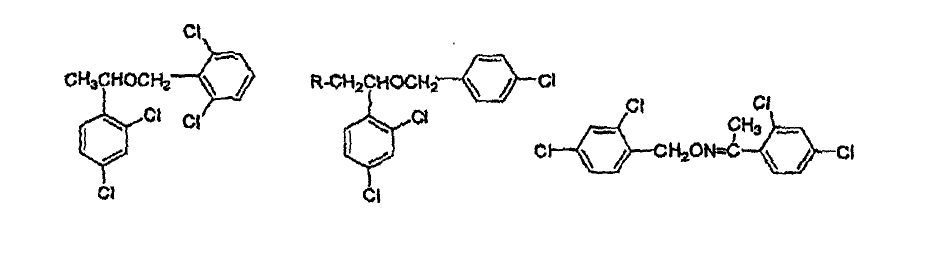 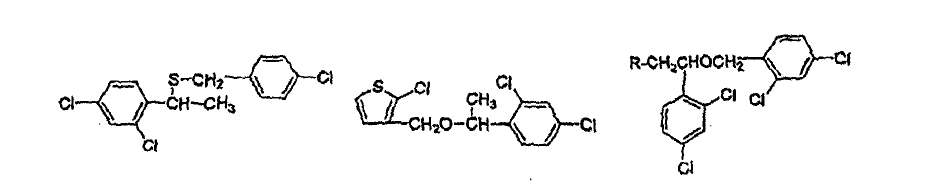 wobei R aus Wasserstoff, Hydroxyl, C1-C3-Alkyl oder C1-C3-Alkoxy besteht.

19. Eine pharmazeutische Zubereitung gemäß Anspruch 18, wobei die Verbindung ausgewählt ist aus der Gruppe:

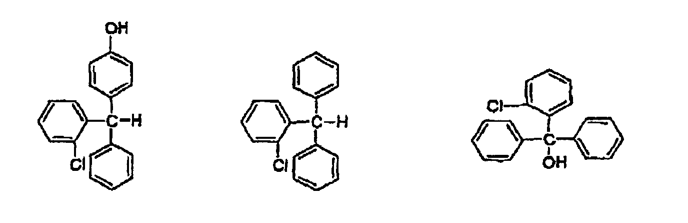

20. Eine pharmazeutische Zubereitung gemäß Anspruch 19, wobei die Verbindung aus 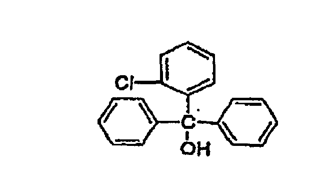 besteht.

21. Eine pharmazeutische Zubereitung gemäß Anspruch 18, wobei die Verbindung aus 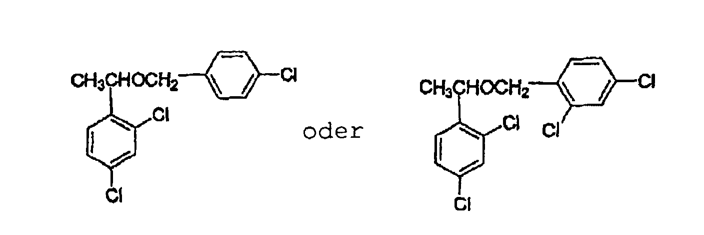 besteht.

## Revendications

1. Composé répondant à la formule : 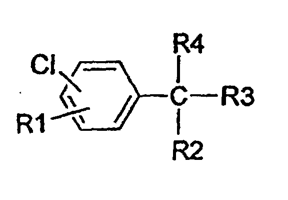 ou un de ses sels pharmaceutiquement acceptables, formule dans laquelle :
R¹ est choisi dans le groupe consistant en l'hydrogène, un groupe hydroxyle et un halogène ;
R² est choisi dans le groupe consistant en néant, l'hydrogène, un groupe phényle et un groupe phényle substitué, le substituant étant un groupe hydroxyle ;
R³ est choisi dans le groupe consistant en l'hydrogène, un groupe hydroxyle, un groupe alkyle en C₁ à C₃ et un groupe alkoxy en C₁ à C₃ ;
R⁴ est choisi dans le groupe consistant en des groupes soufre-CH₂-R⁵, oxygène-CH₂-R⁵, =N-O-CH₂-R⁵, oxygène-phényl-CH=CH₂, CH₂-CH(CH₃)-S-phényle substitué, phényle et phényle substitué, le substituant étant choisi dans le groupe consistant en un groupe hydroxyle et un halogène ;
R⁵ est choisi dans le groupe consistant en des groupes vinyle, phényle, phényle mono-substitué avec un halogène, phényle disubstitué avec un halogène, phényl-S-phényle, CH₂-O-phényle, CH₂-O-phényle substitué avec un halogène, et : 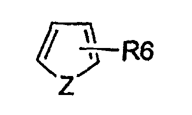
dans lequel Z représente le soufre, l'oxygène ou l'azote ; et R⁶ est choisi dans le groupe consistant en l'hydrogène et un halogène,
destiné à être utilisé dans l'inhibition d'une prolifération cellulaire indésirable chez un mammifère.

2. Composé suivant la revendication 1, ledit composé étant choisi dans le groupe de composés : 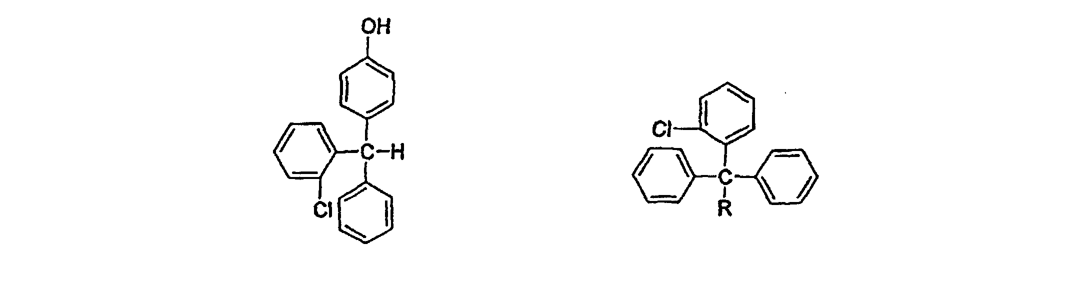 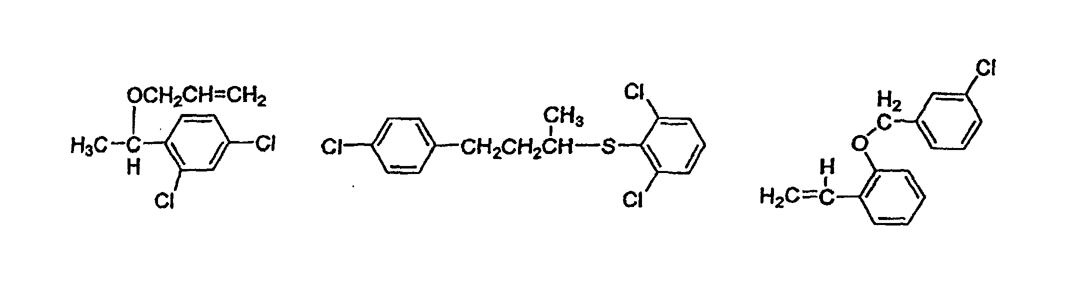 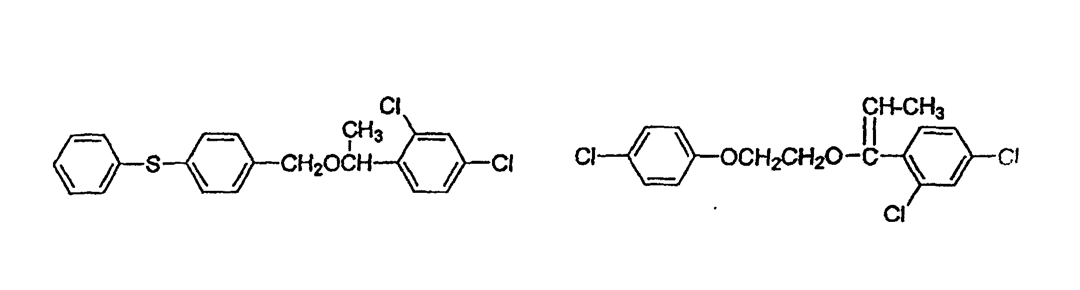 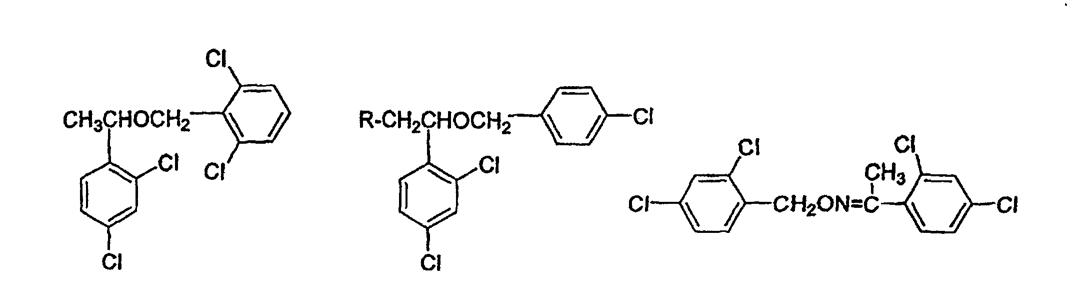 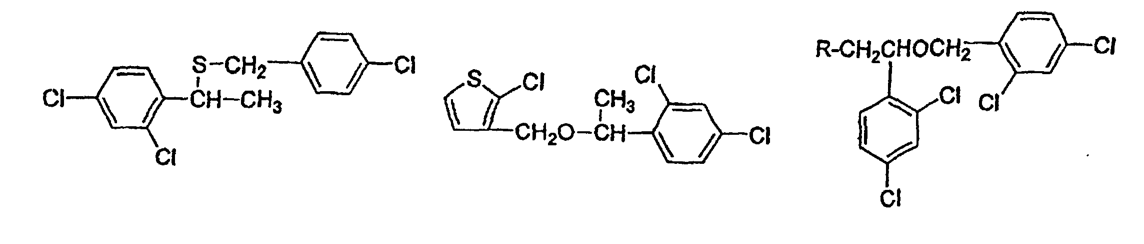 dans lesquels R représente l'hydrogène, un groupe hydroxyle, un groupe alkyle en C₁ à C₃ ou un groupe alkoxy en C₁ à C₃.

3. Composé suivant la revendication 2, ledit composé étant choisi dans le groupe de composés :

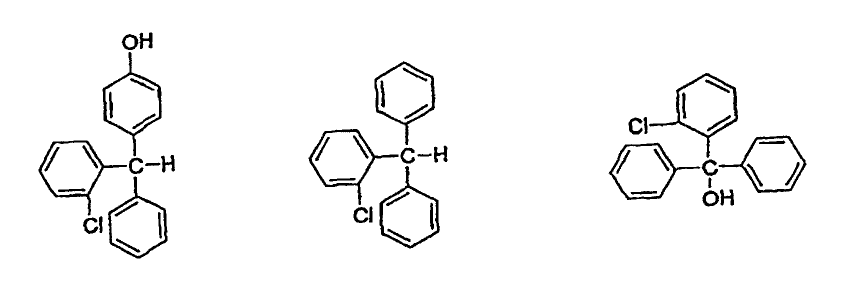

4. Composé suivant la revendication 3, ledit composé étant le suivant :

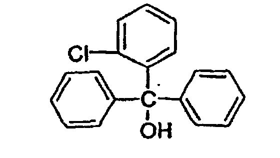

5. Composé suivant la revendication 2, ledit composé étant le suivant :

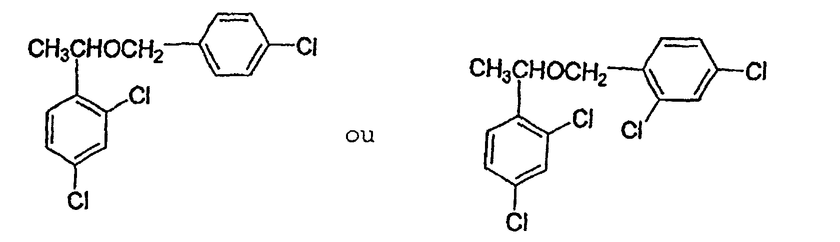

6. Composé suivant l'une quelconque des revendications précédentes, dans lequel les cellules de mammifère sont choisies dans le groupe consistant en des cellules de muscle lisse vasculaire, des fibroblastes, des cellules endothéliales et des cellules cancéreuses.

7. Utilisation d'un composé suivant l'une quelconque des revendications 1 à 5 dans la production d'un médicament destiné au traitement d'une affection **caractérisée par** une prolifération cellulaire indésirable chez un mammifère.

8. Utilisation suivant la revendication 7, dans laquelle la prolifération cellulaire indésirable chez un mammifère est le cancer, un état angiogénique, un état artériosclérotique, une maladie dermatologique, une maladie inflammatoire ou une fibrose.

9. Utilisation suivant la revendication 8, dans laquelle ledit cancer est choisi dans le groupe consistant en : le cancer des voies biliaires ; le cancer du cerveau; le cancer du sein ; le cancer du col ; le choriocarcinome; le cancer du colon ; le cancer de l'endomètre ; le cancer de l'oesophage ; le cancer gastrique ; des néoplasies hématologiques ; des néoplasies intraépithéliales ; le cancer du foie ; le cancer du poumon ; des lymphomes ; des neuroblastomes ; le cancer buccal ; le cancer des ovaires ; le cancer du pancréas ; le cancer de la prostate ; le cancer du rectum ; des sarcomes ; le cancer de la peau ; le cancer des testicules ; le cancer de la thyroïde et le cancer rénal.

10. Utilisation suivant la revendication 9, dans laquelle ledit cancer est le cancer de la peau.

11. Utilisation suivant la revendication 9, dans laquelle ledit cancer est le cancer du colon.

12. Utilisation suivant la revendication 8, dans laquelle ladite affection artériosclérotique est choisie dans le groupe consistant en l'athérosclérose et des complications vasculaires du diabète.

13. Utilisation suivant la revendication 8, dans laquelle ladite affection angiogénique est choisie dans le groupe consistant en : la rétinopathie diabétique ; le glaucome néovasculaire ; l'arthrite rhumatoïde ; un état angiogénique non cancéreux ; des tumeurs solides, un état angiogénique cancéreux ; des hémangioendothéliomes ; des hémangiomes ; le sarcome de Kaposi et le psoriasis.

14. Utilisation suivant la revendication 8, dans laquelle ladite maladie dermatologique est choisie dans le groupe consistant en : des chéloïdes ; des cicatrices hypertrophiques ; la kératose séborrique ; une infection par le virus du papillome ; la kératose actinique et l'eczéma.

15. Utilisation suivant la revendication 8, dans laquelle ladite maladie inflammatoire est choisie dans le groupe consistant en : la glomérulonéphrite proliférative ; le lupus érythémateux ; la sclérodermie ; l'arthrite temporale ; la thromboangiite oblitérante ; le syndrome adéno-cutanéo-muqueux et les léiomyomes utérins.

16. Préparation pharmaceutique comprenant un implant à libération prolongée contenant un composé répondant à la formule : 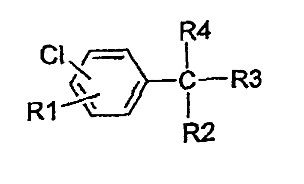 ou un de ses sels pharmaceutiquement acceptables, formule dans laquelle :
R¹ est choisi dans le groupe consistant en l'hydrogène, un groupe hydroxyle et un halogène ;
R² est choisi dans le groupe consistant en néant, l'hydrogène, un groupe phényle et un groupe phényle substitué, le substituant étant un groupe hydroxyle ;
R³ est choisi dans le groupe consistant en l'hydrogène, un groupe hydroxyle, un groupe alkyle en C₁ à C₃ et un groupe alkoxy en C₁ à C₃ ;
R⁴ est choisi dans le groupe consistant en des groupes soufre-CH₂-R⁵, oxygène-CH₂-R⁵, =N-O-CH₂-R⁵, oxygène-phényl-CH=CH₂, CH₂-CH(CH₃)-S-phényle substitué, phényle et phényle substitué, le substituant étant choisi dans le groupe consistant en un groupe hydroxyle et un halogène ;
R⁵ est choisi dans le groupe consistant en des groupes vinyle, phényle, phényle mono-substitué avec un halogène, phényle disubstitué avec un halogène, phényl-S-phényle, CH₂-O-phényle, CH₂-O-phényle substitué avec un halogène, et : 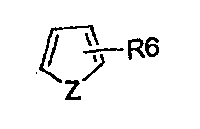
dans lequel Z représente le soufre, l'oxygène ou l'azote ; et R⁶ est choisi dans le groupe consistant en l'hydrogène et un halogène.

17. Préparation pharmaceutique comprenant un cocktail d'agents anti-cancéreux comprenant un composé répondant à la formule : 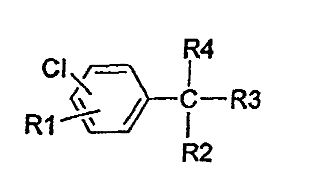 ou un de ses sels pharmaceutiquement acceptables, formule dans laquelle :
R¹ est choisi dans le groupe consistant en l'hydrogène, un groupe hydroxyle et un halogène ;
R² est choisi dans le groupe consistant en néant, l'hydrogène, un groupe phényle et un groupe phényle substitué, le substituant étant un groupe hydroxyle ;
R³ est choisi dans le groupe consistant en l'hydrogène, un groupe hydroxyle, un groupe alkyle en C₁ à C₃ et un groupe alkoxy en C₁ à C₃ ;
R⁴ est choisi dans le groupe consistant en des groupes soufre-CH₂-R⁵, oxygène-CH₂-R⁵, =N-O-CH₂-R⁵, oxygène-phényl-CH=CH₂, CH₂-CH(CH₃)-S-phényle substitué, phényle et phényle substitué, le substituant étant choisi dans le groupe consistant en un groupe hydroxyle et un halogène ;
R⁵ est choisi dans le groupe consistant en des groupes vinyle, phényle, phényle mono-substitué avec un halogène, phényle disubstitué avec un halogène, phényl-S-phényle, CH₂-O-phényle, CH₂-O-phényle substitué avec un halogène, et : 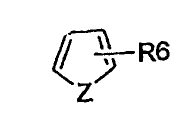
dans lequel Z représente le soufre, l'oxygène ou l'azote ; et R⁶ est choisi dans le groupe consistant en l'hydrogène et un halogène.

18. Préparation pharmaceutique suivant la revendication 16 ou 17, dans laquelle le composé est choisi dans le groupe : 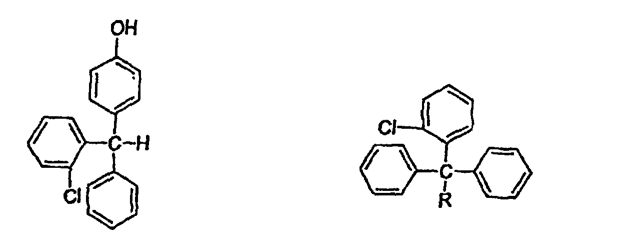 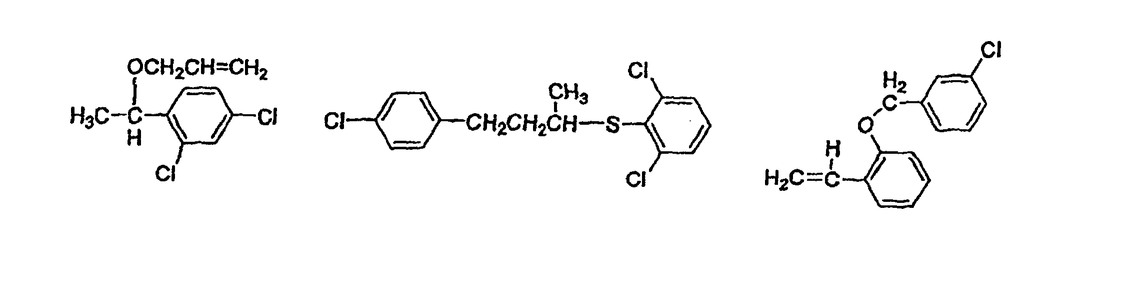 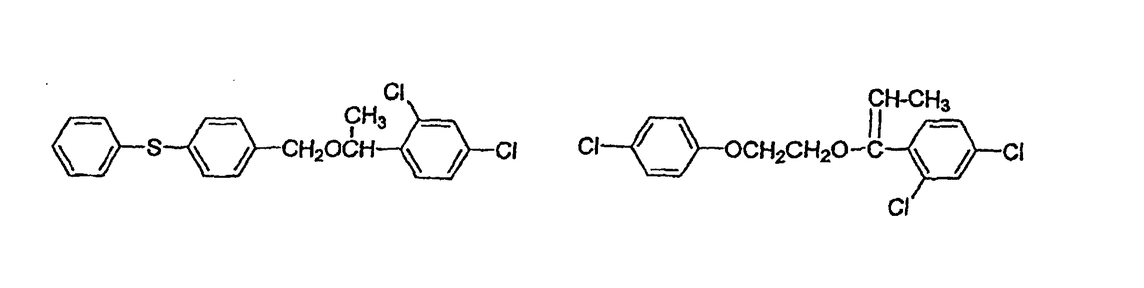 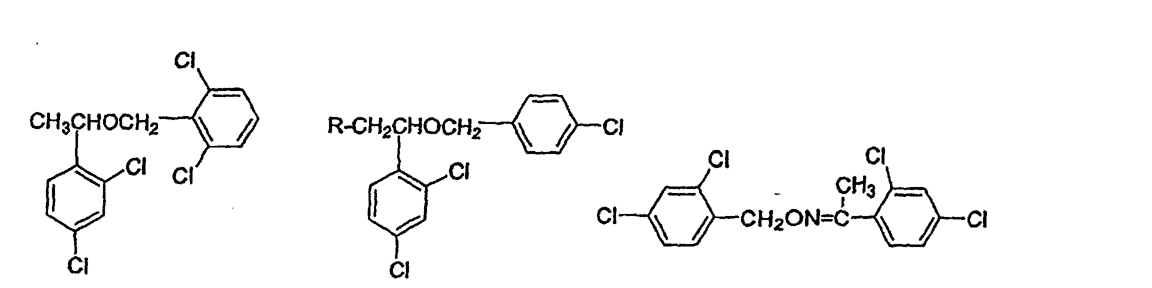 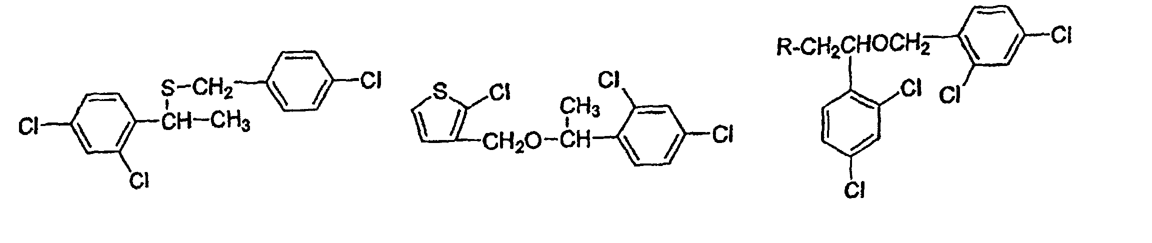 formules dans lesquelles R représente l'hydrogène, un groupe hydroxyle, un groupe alkyle en C₁ à C₃ ou un groupe alkoxy en C₁ à C₃.

19. Préparation pharmaceutique suivant la revendication 18, dans laquelle le composé est choisi dans le groupe :

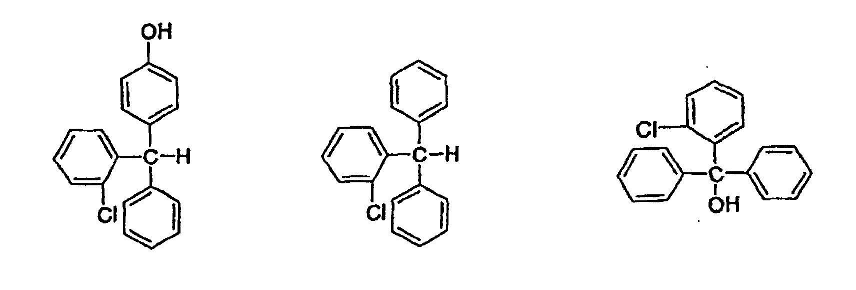

20. Préparation pharmaceutique suivant la revendication 19, dans laquelle le composé est le suivant :

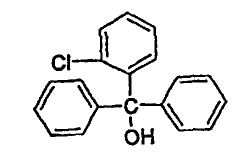

21. Préparation pharmaceutique suivant la revendication 18, dans laquelle le composé est le suivant :
